(19) Europäisches Patentamt
European Patent Office
Office européen des brevets

(11) Numéro de publication: **0 219 407**
**A1**

(12) # DEMANDE DE BREVET EUROPEEN

(21) Numéro de dépôt: 86402117.5

(22) Date de dépôt: 26.09.86

(51) Int. Cl.⁴: **C 12 N 15/00**
C 12 N 9/00, G 01 N 33/68
G 01 N 33/577

(30) Priorité: 27.09.85 FR 8514397

(43) Date de publication de la demande:
22.04.87 Bulletin 87/17

(84) Etats contractants désignés:
AT BE CH DE FR GB IT LI LU NL SE

(71) Demandeur: INSTITUT PASTEUR
25-28, rue du Docteur Roux
F-75724 Paris Cédex 15(FR)

(71) Demandeur: CENTRE NATIONAL DE LA RECHERCHE SCIENTIFIQUE (CNRS)
15, Quai Anatole France
F-75007 Paris(FR)

(72) Inventeur: Leclercq, Roland
21, rue Renoir
F-92700 Colombes(FR)

(72) Inventeur: Brisson, Anne
1, rue de la Pléiade
F-94240 l'Hay-les-Roses(FR)

(72) Inventeur: Courvalin, Patrice
13, rue Emile Duclaux
F-75015 Paris(FR)

(74) Mandataire: Peaucelle, Chantal et al,
S.C. Ernest Gutmann - Yves Plasseraud 67, boulevard Haussmann
F-75008 Paris(FR)

(54) **Fragment d'ADN, comportant au moins une partie d'un gène de résistance aux lincosamides, procédé de préparation et application biochimiques.**

(57) Le fragment de nucléotide de l'invention comprend au moins une partie du gène *lin*A, ce géne codant pour un polypeptide capable d'inactiver les lincosamides et de conférer des propriétés de résistance à la lincomycine, et étant inclus dans la séquence représentée sur la figure 6.

| | | |
|---|---|---|
| GAATTCCTAAG | ACGTTACCAC | |
| ATTGTTTTAC | GTTAAAAGCC | |
| TTTTTAATTT | TTAAAATTTC | 60 |
| CAGTATATCT | GCGTAACTTA | |
| CATTATCAAT | TTTCTTTTCT | |
| CTCCATGGGC | GTTGCTTCCC | 120 |
| ACTTTTCGTC | ATATCTTTTA | |
| ATGTTTCGTT | ATCTTGATTT | |
| TCGTCAATAT | ATTTAGTAGT | 180 |
| ATTATATTGC | ATATAAAAAT | |
| CCCCCAGTTT | TATTTAATGA | |
| TTCGACACCT | TAATAATATA | 240 |
| ACTGGGGTGT | TTTCATGTCA | |
| AATTTTCTTT | TCAACCACAA | |
| AAATACATAA | AAACACTGTC | 300 |
| ACATCAACGA | TAAATCATAT | |
| TTTTAAAAAA | GAACGTTATT | |
| TCTATATAGT | ATCAAGACAA | 360 |
| GAAGAAACTC | GTTTTCAACT | |
| CGTTTCAAAT | TCCCAAGTTA | |
| GGAGGGATAAA | ATG AAA AAT | 420 |

FIG.6A

./...

EP 0 219 407 A1

FIG.6B

AAT AAT GTA ACA GAA AAA
GAA TTA TTT TAT ATT TTA
GAT TTA TTT GAA CAC ATG
AAA GTA 480

ACT TAT TGG TTA GAT GGT
GGC TGG GGG GTA GAT GTA
TTA ACT GGA AAA CAA CAA
AGA GAA 540

CAC AGA GAT ATA GAT ATA
GAT TTT GAC GCT CAA CAC
ACT CAA AAA GTT ATA CAA
AAA TTA 600

GAA GAT ATA GGA TAC AAA
ATA GAA GTT CAT TGG ATG
CCT TCA CGT ATG GAA CTT
AAG CAT 660

GAA GAA TAT GGG TAT TTA
GAT ATT CAT CCT ATA AAT
CTA AAT GAT GAT GGA TCA
ATT ACC 720

FIG.6C

CAA GCA AAC CCA GAA GGT
GGT AAT TAT GTT TTC CAA
AAT GAC TGG TTT TCA GAA
ACT AAT 780

TAC AAA GAT CGA AAA ATA
CCA TGT ATT TCA AAA GAA
GCT CAA CTT CTT TTT CAT
TCT GGT 840

TAT GAT TTA ACA GAA ACA
GAC CAT TTT GAT ATA AAA
AAT TTA AAA TCA ATA ACA

TAA TTG 900

CTTTATTCCA ATTGCTTTAT
TGACGTTGAG CCTCGGAACC
CTTAACAATC CCAAAACTTG 960

TCGAATGGTC GGCTTAATAG
CTCACGCTAT GCCGACATTC
GTCTGCAAGT TTAGTTAAGG 1020

GTTCTTCTCA ACTTCAATAA
ATTTTCTCGG CATAAATGCG
TGTTCTTTTT TGTCTACTTA 1080

GTAGAC 1086

"FRAGMENT D'ADN, COMPORTANT AU MOINS UNE PARTIE D'UN GENE DE RESISTANCE AUX LINCOSAMIDES, PROCEDE DE PREPARATION ET APPLICATIONS BIOCHIMIQUES"

L'invention est relative à un fragment d'ADN comportant au moins une partie d'un gène de résistance aux lincosamides et à son procédé d'obtention.

Elle vise également ses applications biochimiques, notamment, aux fins de détection de la résistance aux lincosamides dans des bactéries.

La famille des lincosamides est constituée de deux antibiotiques structuralement apparentés, la lincomycine et son dérivé chloré, la clindamycine.

Ces antibiotiques sont largement utilisés en clinique humaine et animale, notamment, pour le traitement des infections à Gram-positif dues, par exemple, à des staphylocoques et à des streptocoques et de certaines infections à Gram-négatifs provoquées, par exemple, par Haemophilus. Ils sont également prescrits dans le cas d'infections dues à des bactéries anaérobies.

Leur utilisation massive a provoqué ces dernières années l'émergence de souches résistantes, en particulier, de staphylocoques et de streptocoques.

La résistance des staphylocoques aux lincosamides est presque toujours associée à une résistance aux macrolides et aux streptogramines de type B (phénotype $MLS_B$). Le mécanisme de cette résistance à large spectre comme l'a montré Weisblum dans Microbiology, p. 199-206, 1974, American Society for microbiology, Washington, D.C. Schlessinger (ed), implique une modification de la cible intracellulaire de l'antibiotique par N-mono ou diméthylation de l'ARN ribosomal 23S.

En se basant sur ce mécanisme de résistance $MLS_B$

très fréquent dans la nature, les bactériologistes médicaux incluent dans leur antibiogramme seulement un ou deux macrolides, puis infèrent la réponse pour l'ensemble des MLS.

Or, de nouveaux types de résistance ont été récemment détectés dans des souches de staphylocoques.

Ainsi, en 1980, deux types de résistance à la lincomycine, différents du phénotype $MLS_B$ ,ont été décrits chez des staphylocoques d'origine animale. Pour le premier, détecté chez S. aureus et S. intermedius, les cellules inactivent la lincomycine et sont sensibles à la clindamycine mais ne sont hautement résistantes qu'à la lincomycine(concentration minimale inhibitrice (CMI) de 64mg/l). Le second type de résistance a été observé chez S. aureus et S.hyicus. Les souches ont un bas niveau de résistance à la lincomycine (CMI de 2 à 4 mg/l) et aux streptogramines de type A en l'absence apparente d'inactivation.

Récemment, les inventeurs ont isolé en clinique humaine une nouvelle souche de staphylocoques, Staphylococcus haemolyticus BM 4610, résistante à la lincomycine et apparemment sensible à la clindamycine, et également sensible aux macrolides et aux streptogramines A et B. Cette souche a été déposée à la Collection nationale de Culture de microorganismes (CNCM) de l'Institut Pasteur le 25 septembre 1985 sous le No I-484. Les études d'inactivation ont mis en évidence une dégradation de la lincomycine et de la clindamycine bien que la CMI de ce dernier produit reste basse.

En ce qui concerne la détection de la résistance aux antibiotiques, l'existence de ce caractère nouveau par rapport au phénotype $MLS_B$ rend une lecture interprétative en utilisant un ou deux macrolides inadéquate.

Il s'ensuit des erreurs possibles d'appréciation avec pour conséquence des erreurs de prescription.

En effet, en réalisant, par exemple, l'antibiogramme avec un disque d'un macrolide tel que l'érythromycine, on peut conclure en faveur d'un phénotype sensible aux MLS qui peut entraîner une prescription de lincomycine alors que la souche peut être résistante à cet antibiotique par inactivation.

On conçoit donc l'intérêt de disposer de nouveaux moyens permettant de déterminer les antibiotiques qui peuvent être prescrits au niveau médical.

En poursuivant leurs recherches sur Staphylococcus haemolyticus BM 4610, les inventeurs ont mis en évidence que la résistance à la lincomycine fait intervenir un plasmide déterminé.

Les développements de ces travaux ont permis de réaliser l'expression de cette résistance chez une autre souche bactérienne et de déterminer la séquence nucléotidique de la région codant pour la résistance à la lincomycine.

L'invention a donc pour but de fournir un fragment d'ADN comportant au moins une partie de la séquence génique capable de conférer des propriétés de résistance aux lincosamides à un microorganisme, plus particulièrement à une bactérie.

Elle vise également en tant que produits intermédiaires, les vecteurs recombinants comportant cette séquence, ainsi que les souches transformées par de tels réplicons.

Selon un autre aspect, l'invention a également pour but de fournir un procédé d'obtention de ce fragment d'ADN par clonage dans une bactérie sensible aux MLS.

Selon encore un autre aspect, elle vise les applications biochimiques de la nouvelle séquence nucléotidique. En particulier, l'invention vise à fournir des sondes permettant de détecter la résistance aux lincosamides chez les bactéries. Elle vise également à fournir le polypeptide codé par la séquence du fragment considéré et les anticorps, plus spécialement les anticorps monoclonaux capables de reconnaître spécifiquement le polypeptide.

Le fragment d'ADN de l'invention est caractérisé en ce qu'il comprend au moins une région du gène codant pour un polypeptide capable d'inactiver les lincosamides et de conférer des propriétés de résistance à la lincomycine. Cette région comporte ou est constituée par les codons du gène <u>lin</u>A, ce gène étant inclus dans la séquence nucléotidique suivante :

```
GAATTCCTAAG ACGTTACCAC ATTGTTTTAC GTTAAAAGCC TTTTTAATTT TTAAAATTTC   60
 CAGTATATCT GCGTAACTTA CATTATCAAT TTTCTTTTCT CTCCATGGGC GTTGCTTCCC  120
 ACTTTTCGTC ATATCTTTTA ATGTTTCGTT ATCTTGATTT TCGTCAATAT ATTTAGTAGT  180
 ATTATATTGC ATATAAAAAT CCCCCAGTTT TATTTAATGA TTCGACACCT TAATAATATA  240
 ACTGGGGTGT TTTCATGTCA AATTTTCTTT TCAACCACAA AAATACATAA AAACACTGTC  300
 ACATCAACGA TAAATCATAT TTTTAAAAAA GAACGTTATT TCTATATAGT ATCAAGACAA  360

GAAGAAACTC GTTTTCAACT CGTTTCAAAT TCCCAAGTTA GGAGGGATAAA ATG AAA AAT  420

AAT AAT GTA ACA GAA AAA GAA TTA TTT TAT ATT TTA GAT TTA TTT GAA CAC ATG AAA GTA  480

ACT TAT TGG TTA GAT GGT GGC TGG GGG GTA GAT GTA TTA ACT GGA AAA CAA CAA AGA GAA  540

CAC AGA GAT ATA GAT ATA GAT TTT GAC GCT CAA CAC ACT CAA AAA GTT ATA CAA AAA TTA  600

GAA GAT ATA GGA TAC AAA ATA GAA GTT CAT TGG ATG CCT TCA CGT ATG GAA CTT AAG CAT  660

GAA GAA TAT GGG TAT TTA GAT ATT CAT CCT ATA AAT CTA AAT GAT GAT GGA TCA ATT ACC  720

CAA GCA AAC CCA GAA GGT GGT AAT TAT GTT TTC CAA AAT GAC TGG TTT TCA GAA ACT AAT  780

TAC AAA GAT CGA AAA ATA CCA TGT ATT TCA AAA GAA GCT CAA CTT CTT TTT CAT TCT GGT  840

TAT GAT TTA ACA GAA ACA GAC CAT TTT GAT ATA AAA AAT TTA AAA TCA ATA ACA TAA TTG  900
 CTTTATTCCA ATTGCTTTAT TGACGTTGAG CCTCGGAACC CTTAACAATC CCAAAACTTG  960
 TCGAATGGTC GGCTTAATAG CTCACGCTAT GCCGACATTC GTCTGCAAGT TTAGTTAAGG 1020
 GTTCTTCTCA ACTTCAATAA ATTTTCTCGG CATAAATGCG TGTTCTTTTT TGTCTACTTA 1080
 GTAGAC                                                            1084
```

L'expression "au moins une partie du gène *lin*A" désigne toute séquence nucléotidique dont la structure correspond à une partie de ce gène, présentant une longueur suffisante pour reconnaître spécifiquement ce gène dans un prélèvement à l'étude, dans des essais d'hybridation mettant en jeu cette séquence en tant que sonde. Cette expression désigne également toute séquence nucléotidique hybridable avec la précédente, telle qu'obtenue par transcription enzymatique inverse de l'ARN correspondant ou encore par synthèse chimique.

Il va de soi que les bases de la séquence nucléotidique considérée peuvent être dans un ordre différent de celui trouvé dans les gènes et/ou que ces bases peuvent être le cas échéant substituées, dès lors qu'une sonde élaborée à partir d'une telle séquence donne une réponse caractéristique et non équivoque quant à la capacité de reconnaître la présence du gène *lin*A.

La séquence codante du gène *lin*A est caractérisée en ce qu'elle est définie par le codon d'initiation ATG en position 413 et le codon de terminaison TAG en position 895 dans une phase de lecture ouverte (ou PLO en abrégé) de 482 paires de base.

Le gène *lin*A est également caractérisé en ce que son promoteur se trouve en position 154.

Le gène *linA* est caractérisé en outre par une teneur en guanine et cytosine de 29%.

Selon un autre aspect, le gène *linA* est caractérisé en ce qu'il est porté par un plasmide de 2,5 kb environ, à savoir pIP855.

Selon encore un autre aspect, le gène *linA* est caractérisé en ce qu'il est porté par un fragment d'ADN de 1,1 kb environ, tel qu'obtenu par digestion à l'aide des endonucléases de restriction *Eco*RI et *Acc*I du plasmide recombinant pAT22, lui-même obtenu par digestion, par *Eco*RI, de pBR329 de 4,1 kb et de pIP855, puis religation.

L'expression de la séquence nucléotidique du déterminant de résistance aux lincosamides, selon l'invention, dans des minicellules d'*E.coli* selon les techniques habituelles, conduit à une protéine ayant une masse moléculaire apparente $\underline{M}_r$ de l'ordre de 19.000 à 21.000 daltons, telle que déterminée par électrophorèse sur gel de polyacrylamide-SDS.

Cette estimation de $\underline{M}_r$ est en accord avec la $\underline{M}_r$ de 19.020 daltons déduite de la séquence nucléotidique de la PLO de 482pb ci-dessus.

La séquence correspondante d'acides aminés est la suivante :

Met Lys Asn

Asn Asn Val Thr Glu Lys Glu Leu Phe Tyr Ile Leu Asp Leu Phe Glu His Met Lys Val

Thr Tyr Trp Leu Asp Glv Gly Trp Gly Val Asp Val Leu Thr Gly Lys Gln Gln Arg Glu

His Arg Asp Ile Asp Ile Asp Phe Asp Ala Gln His Thr Gln Lys Val Ile Gln Lys Leu

Glu Asp Ile Gly Tyr Lys Ile Glu Val His Trp Met Pro Ser Arg Met Glu Leu Lys His

Glu Glu Tyr Gly Tyr Leu Asp Ile His Pro Ile Asn Leu Asn Asp Asp Gly Ser Ile Thr

Gln Ala Asn Pro Glu Gly Gly Asn Tyr Val Phe Gln Asn Asp Trp Phe Ser Glu Thr Asn

Tyr Lys Asp Arg Lys Ile Pro Cys Ile Ser Lys Glu Ala Gln Leu Leu Phe His Ser Gly

Tyr Asp Leu Thr Glu Thr Asp His Phe Asn Ile Lys Asn Leu Lys Ser Ile Thr

En tant qu'intermédiaires, l'invention vise également les vecteurs recombinants, notamment les plasmides hybrides comportant au moins une partie du gène linA.

D'autres intermédiaires selon l'invention comprennent les souches bactériennes modifiées, ou transformants, caractérisées par la présence d'au moins une partie du gène linA conférant un phénotype Gots positif.

Les transformants renfermant le gène linA expriment la résistance aux lincosamides et sont capables de les inactiver.

Le même phénotype de résistance par inactivation a été détecté chez d'autres souches isolées en clinique humaine, telles que Staphylococcus aureus, Staphylococcus epidermidis, Staphylococcus cohnii et Streptococcus faecium.

Conformément à l'invention, la séquence nucléotidique comportant ou constituée par la région codant pour un polypeptide capable d'inactiver la lincomycine est obtenue par :

- digestion de pAT22 par les endonucléases EcoRI et AccI et religation, ce qui conduit au plasmide recombinant pAT151 comportant un fragment d'ADN de pIP855 de 1,1kb environ,

- clonage du fragment EcoRI-AccI de 1,1kb de pAT151 dans pUC8 de 2,75 kb avec digestion de pUC8 par AccI et EcoRI et ligation,

- purification du fragment de 1,1kb, par sous-clonage à partir de pAT152 digéré par EcoRI et PstI,

- clonage dans une entérobactérie sensible aux antibiotiques MLS, plus spécialement une souche mutante de E.coli sensible aux MLS, en particulier E.coli DB10, en sélectionnant en particulier pour la résistance à l'ampicilline et à la clindamycine et en vérifiant le phénotype de Gots positif des transformants obtenus.

L'invention vise, en outre, les applications biochimiques de la séquence ou d'une partie de la séquence du gène <u>linA</u> définie ci-dessus.

Ces applications comprennent l'élaboration de sondes pour la détection de la résistance aux lincosamides dans les bactéries.

Le fragment d'ADN utilisé présente alors un nombre de nucléotides suffisant pour obtenir la spécificité requise et la formation d'un hybride stable. Un tel fragment renferme au minimum environ 14 nucléotides.

Des sondes appropriées pour ce type de détection sont avantageusement marquées par un élément radioactif ou tout autre groupe permettant sa reconnaissance à l'état hybridé avec la préparation renfermant l'ADN à étudier.

Selon les techniques classiques, ces sondes sont mises en contact avec un échantillon biologique renfermant des bactéries, ou directement avec ces bactéries ou leurs acides nucléiques, dans des conditions autorisant l'hybridation éventuelle de la séquence nucléotidique de la sonde avec une séquence complémentaire, éventuellement contenue dans le produit testé.

On peut, par exemple, mettre en oeuvre la méthode d'hybridation sur taches. Cette méthode comporte, après dénaturation de l'ADN préalablement obtenu à partir de bactéries provenant de selles humaines, le dépôt d'une quantité aliquote de cet ADN sur des membranes de nitrocellulose, l'hybridation de chaque membrane dans les conditions usuelles avec la sonde et la détection, de manière classique, de l'hybride formé.

On peut aussi utiliser une méthode d'hybridation sur réplique, selon la technique de Southern. Cette méthode comprend la séparation électrophorétique en gel d'agarose des fragments d'ADNs engendrés après traitement de l'ADN par des enzymes de restriction, le transfert après dénaturation alcaline sur des membranes appropriées et leur hybridation avec la sonde dans les conditions usuelles. Il n'est pas toujours nécessaire de procéder à l'expression préalable de l'ADN. Il suffit que l'ADN soit rendu accessible à la sonde.

L'invention fournit ainsi les moyens de détecter la présence chez les bactéries de gènes identiques au gène linA ou homologues.

La distribution du gène a été ainsi étudiée par dot-blot chez des souches de streptocoques et de staphylocoques.

La reproductibilité de la détection a été testée en utilisant une sonde intragénique contenant un insérat de 77 paires de bases, prélevé entre les sites Sau3A-Sau3A du gène linA et en réalisant des essais d'hybridation sur des colonies provenant de bactéries pathogènes.

Les fragments intragéniqued sont avantageusement utilisés comme sondes pour l'étude de la dissémination de la résistance aux lincosamides. Les hybridations avec l'ADN d'autres cocci à Gram-positifs permettent d'étudier la variabilité de la résistance.

Selon encore d'autres applications biochimiques on utilise le polypeptide codé par le gène linA pour le dosage de lincosamides isolés ou associés avec d'autres antibiotiques et pour modifier chimiquement les lincosamides en vue par exemple d'hémisynthèse.

L'invention vise également les applications immunologiques du peptide codé, plus spécialement pour l'élaboration d'antisérum spécifique ainsi que d'anticorps polyclonaux et, en particulier d'anticorps monoclonaux. Les anticorps polyclonaux sont formés selon les techniques classiques par injection du polypeptide à des animaux, récupération des antisérums, puis des anticorps à partir des antisérums par exemple par chromatographie d'affinité.

D'autres avantages et caractéristiques de l'invention sont rapportées dans la description qui suit concernant le clonage du gène _linA_ chez _E.coli_, la détermination de sa séquence nucléotidique et l'analyse des produits d'expression. Dans cette description, il sera fait référence aux figures 1 à 7,

- la figure 1 représentant le schéma de construction des plasmides pAT151 et pAT152,
- la figure 2, l'analyse par endonucléases de restriction des plasmides hybrides pAT22, pAT151 et pAT152,
- la figure 3, la réponse au test de Gots de différentes souches étudiées,
- la figure 4, un autoradiogramme des polypeptides marqués à la $^{35}S$-méthionine analysés en minicellules,
- la figure 5, la stratégie de séquence adoptée pour séquencer l'insérat de 1,1kb dont question dans la figure 1,
- la figure 6, la séquence nucléotidique du fragment _Eco_RI-_Acc_I de 1086 pb, avec indication de la séquence d'amino-acides codés,
- la figure 7, la représentation graphique des codons d'arrêt de la traduction dans les trois cadres de lecture des deux brins d'ADN séquencés, et
- la figure 8, la structure secondaire proposée en aval du gène _linA_.

Les références bibliographiques mentionnées dans ce qui suit sont données en fin de description.

## Matériel et méthodes

I Souches bactériennes.

Les origines et les propriétés des souches bactériennes utilisées sont résumées dans le tableau 1 suivant (*E.coli* étant naturellement résistant aux macrolides, lincosamides et streptogramines, on utilise la souche d'*E.coli* DB10 qui est un mutant de perméation à ces antibiotiques (1).

TABLEAU 1

| BACTERIE | SOUCHE | CARACTERES (a) |
|---|---|---|
| *Staphylococcus haemolyticus* | BM4610 | LnPnKm |
| *Escherichia coli* | DB10 | Dérivé de PR7($F^-$ *thi* *leu* *rus* *pup* *nal-r*)sensible aux MLS |
| | K-12 JM101 | (*lac-pro*),*thr*, *supE*, F'*traD* 36, *proAB* +,*lacI*$^q$ |
| | K-12 AR1062 | *thr*,*leu*,*lac*,*gal*, *xyl*,*mal*,*mtl*, *hsdS* |

(a) Les symboles génétiques sont d'après Bachmann et coll.(2) et Novick et coll.(3).

2. MILIEUX DE CULTURE

Les souches d'*E.coli* et de *S.haemolyticus* sont cultivées en milieu coeur-cerveau (Difco) bouillon ou agar. On réalise les antibiogrammes sur milieu de Mueller-Hinton- (Diagnostic Pasteur) agar avec des disques imprégnés d'antibiotiques (Diagnostics Pasteur). Toutes les incubations sont effectuées à 37°C.

3. TESTS DE GOTS(4)

Principe : si l'on cultive une souche productrice d'une enzyme inactivant l'antibiotique sur une gélose ensemencée avec une souche indicatrice et contenant une quantité d'antibiotique juste suffisante pour inhiber cette dernière, la production d'enzyme provoquera une dégradation de l'antibiotique dans le milieu de culture et se traduira par une croissance de kla souche indicatrice autour de la souche productrice.

Les tests ont été réalisés sur milieu coeur-cerveau contenant la couche indicatrice <u>Sarcina</u> <u>lutea</u> ATCC 9341 et de la clindamycine (0,5 mg/l). Les incubations ont été faites à 30°C pendant 48 h.

4. PLASMIDES

Les plasmides utilisés dans ces travaux sont énumérées dans le tableau 2.

Tableau 2.

| Plasmide | Caractères phénotypiques(a) | Origine |
|---|---|---|
| pUC8 | $Tra^-$, $Mob^-$, Ap | |
| pIP855 | $Tra^-$, Cl, Ln | plasmide naturel |
| pAT22 | $Tra^-$, $Mob^-$, Ap Cl Ln Tc | pBR329 pIP855 EcoRI |
| pAT151 | $Tra^-$, $Mob^-$, Ap CI Ln | |
| pAT152 | $Tra^-$, $Mob^-$, Ap CI Ln | pUC8 pIP855 EcoRI-AccI-1,1kb |

(a) La nomenclature des caractéres phénotypiques des plasmides est donnée d'aprés Novick et coll.(3).

5. PREPARATION DE L'ADN PLASMIDIQUE

· Préparation rapide : On effectue la caractérisation des plasmides recombinants obtenus après transformation après extraction de l'ADN plasmidique selon la technique de Birnboim et Doly (5) par digestion par endonucléases de restriction.

· Obtention d'ADN purifié : On extrait l'ADN plasmidique par la technique de lyse alcaline (6) puis on purifie par centrifugation à l'équilibre en gradient de chlorure de cesium-bromure d'éthidium.

6. METHODES D'ANALYSE DES ADN.

a- Coupure par endonucléases de restriction :

L'arrêt de la réaction enzymatique est effectué par dénaturation thermique ou par extraction au phénol selon les enzymes.

b- Electrophorèse analytique ou préparative :

Les électrophorèses analytiques et préparatives sont réalisées en gels d'agarose à 0,8% immergés dans du tampon Tris-borate-EDTA (Tris base 45mM,acide borique 45mM,EDTA 1,25mM) en présence de bromure d'éthidium (0,5mg/l).

Les fragments d'ADN sont purifiés par électrotransfert sur une membrane de dialyse.

7. EXPRESSION DANS DES MINICELLULES D'E.COLI

La souche d'*E.coli* AR1062 (7) est transformée avec l'ADN des plasmides à étudier et cultivée une nuit sur milieu coeur-cerveau. Les minicellules sont purifiées par centrifugation en gradient de sucrose . Les protéines synthétisées dans les minicellules sont marquées radioactivement par incorporation de ($^{35}$S)-méthionine. Leur masse moléculaire est déterminée par électrophorèse en gel de polyacrylamide à 12,5% en présence de dodécyl-sulfate de sodium (SDS).

8. SEQUENCAGE DE L'ADN

Les fragments d'ADN à séquencer sont clonés dans les formes réplicatives des bactériophages M13mp18,M13mp19 ou M13mp10 et introduits par transformation dans la souche d'*E. coli* JM101. Les bactériophages recombinants sont isolés sur gélose molle coeur-cerveau en présence d'isopropyl-β-D-thiogalactopyranoside (80 mg/l) et de 5-bromo-4-chloro-3-indolyl-β-D-galactopyranoside (40mg/l). Les formes simple brin sont purifiées suivant le protocole décrit par Amersham.

La séquence est déterminée par la technique de terminaison de chaîne (8).Les fragments sont marqués radioactivement par incorporation d'($\alpha^{32}$P)-déoxy ATP. Les échantillons sont déposés sur des gels de polyacrylamide à 6 ou 8% de 0,35 mm d'épaisseur en tampon Tris-borate-EDTA (Tris base 90mM,acide borique 90mM,EDTA 2,5mM).

9. ENZYMES ET REACTIFS

Les enzymes utilisées et l'($\alpha^{32}$P)-dATP proviennent d'Amersham,les phages M13mp18, mp19 et mp10 de PL-Biochemicals, les antibiotiques des Laboratoires suivants : ampicilline (Bristol),tétracycline (Pfizer), lincomycine et clindamycine (Upjohn).

EXEMPLE I :

1. CLONAGE DU GENE DE RESISTANCE AUX LINCOSAMIDES CHEZ E.COLI

a.-Construction des plasmides pAT151 et pAT152

La construction de ces plasmides est illustrée sur la figure 1

Les tailles des différents fragments sont indiquées en kilobases. Les flèches en pointillés symbolisent les gènes de résistance inactivés par le clonage.

Dans les plasmides,Ap désigne la résistance à l'ampicilline Cm au chloramphénicol, Tc à la tétracycline et ORI l'origine de réplication.

On procède comme suit :

A) construction du plasmide pAT22

On soumet les plasmides pBR329 de 4,1kb et pIP855 de 2,5kb à une étape de digestion par *Eco*RI puis de ligation.

On obtient le plasmide pAT22.

B) obtention du plasmide pAT151: On soumet pAT22 à une digestion par les endonucléases *Eco*RI et *Acc*I et à une religation. On obtient le plasmide recombinant pAT151 qui a perdu le petit fragment *Eco*RI-*Acc*I (800pb) de pBR329 et le grand fragment *Eco*RI-*Acc*I (1,4 kb) de pIP855. Le fragment de pIP855 conservé (1,1 kb) confère la résistance aux lincosamides.

C) obtention du plasmide pAT152

Le plasmide pUC8, qui renferme le gène de résistance à l'ampicilline, est soumis à une opération de digestion à l'aide de *Acc*I et *Eco*RI, puis on effectue une ligation avec le fragment *Eco*RI-*Acc*I de 1,1 kb de pAT151, ce qui conduit au plasmide pAT152.

Le fragment de 1,1 kb est purifié pour séquençage à partir de pAT152. A cet effet, on soumet pAT152 à une digestion par les enzymes EcoRI et PstI.(AccI peut être utilisée à la place de PstI mais nécessite des sels d'incubation spéciaux, qui donnent lieu à des digestions partielles fréquentes et coûte très cher.)

Les clonages sont effectués dans E.coli DB10 en sélectionnant pour la résistance à l'ampicilline (100g/l) et la clindamycine (1g/l) et en vérifiant le phénotype Gots positif des transformants obtenus. Les constructions plasmidiques sont vérifiées par digestion par les endonucléases de restriction appropriées.

On a reporté sur la figure 2 les résultats de l'analyse par les endonucléases de restriction des plasmides hybrides pAT22, pAT151 et pAT152. Les nombres 1 à 8 correspondent respectivement aux fragments suivants :

1. λ/HindIII-EcoRI
2. pAT22/EcoRI
3. pBR329/EcoRI
4. pBR329/AccI-EcoRI
5. pAT22/AccI-EcoRI
6. pAT151/AccI-EcoRI
7. pAT152/PstIEcoRI
8. pUC8/PstI-EcoRI
9. λ/hindIII-EcoRI

Les fragments de 2,5 kb (pIP 855 EcoRI) et de 1,1kb (EcoRI-AccI) sont indiqués par des flèches.

b- Phénotypes conférés par ces plasmides :

.Concentrations minimales inhibitrices (CMI).

Les CMI en mg/ml de la clindamycine et de la lincomycine vis-à-vis de S. haemolyticus, d'E.coli DB10 et d'*E.coli* DB10 hébergeant les plasmides pAT22,pAT151 et pAT152 sont indiquées dans le tableau 3 ci-après.

| Souche | **Lincomycine** | **Clindamycine** |
|---|---|---|
| *S.haemolyticus* BM4610 | 64 | 0,12 |
| *E.Coli* DB10 | 8 | 0,5 |
| *E.Coli* DB10/pAT22* | 16 | 4 |
| *E.Coli* DB10/pAT151 | 32 | 4 |
| *E.Coli* DB10/pAT152 | 64 | 32 |

* cette souche a été déposée à la Collection nationale de culture des microorganismes, à l'Institut Pasteur le 25 septembre 1985 sous le No I-485.

.Test de Gots

Les quatre souches BM4610, DB10/pAT22, DB10/pAT151 et DB10/pAT152 présentent un test de Gots positif. Les résultats obtenus sont rapportés sur la figure 3 où 1 à 5 désignent respectivement :

1. *E.coli* DB10
2. *E.coli* DB10 / pAT22
3. *E.coli* DB10 / pAT151
4. *E.coli* DB10 / pAT152
5. *S.haemolyticus*

2. ANALYSE DU PRODUIT DU GENE DE RESISTANCE DANS DES MINICELLULES D'E.COLI

Les polypeptides codés par les plasmides pUC8, pAT151 et pAT152 sont visualisés sur gel de polyacrylamide.Les résultats obtenus sont représentés sur la figure 4 qui correspond à un autoradiogramme des polypeptides marqués à la $^{35}$S-méthionine analysés en minicellules. La bande A correspond à celle de la protéine de résistance aux lincosamides.

La bande (C) correspondant à la bêta-lactamase TEM-1 (28.500 daltons) qui confère la résistance à l'ampicilline est commune aux trois plasmides. On distingue au-dessus la bande (D) correspondant au précurseur de cet enzyme (31 000 daltons).

Les plasmides pAT151 et pAT152 codent pour un produit correspondant à une bande supplémentaire (A) de taille estimée 21 000 daltons. Cette bande correspond à la protéine de résistance aux lincosamides.

Le plasmide pAT151 présente de plus une bande (B) qui correspond à la chloramphénicol-acétyl-transférase (résistance au chloramphénicol) tronquée par le clonage EcoRI-AccI dans pBR329 (23000 daltons).

3. SEQUENCE NUCLEOTIDIQUE DU FRAGMENT DE 1,1KB

a - Sous-clonage dans le bactériophage M13. Stratégie de séquence.

Le fragment AccI-EcoRI ou PstI-EcoRI de 1,1 kb purifié est digéré par plus de vingt endonucléases de restriction, dont AluI,HaeIII,HpaII,RsaI,Sau3A, et TaqI.Parmi les enzymes utilisées seules AluI, Sau3A, et TaqI possèdent des sites de reconnaissance. La stratégie de séquence présentée sur la figure 5, basée sur ces résultats combine l'utilisation d'endonucléases et celle de l'exonucléase BaI31.

Sur cette figure, chaque bande horizontale représente la carte de restriction de l'endonucléase indiquée à gauche. Les sites d'insertion dans l'ADN du phage M13 sont indiqués entre parenthèses. Les flèches indiquent la direction et l'étendue de la réaction de séquence. Le gène de structure linA est représenté par une flèche ondulée.

Les bactériophages recombinants obtenus après clonage du fragment entier ou digéré par les enzymes AluI, Sau3A et TaqI permettent de déterminer la séquence de la quasi-totalité de l'insert de 1,1 kb (fig.5).

Le reste de la séquence et les zones de chevauchements nécessaires à l'agencement des différents fragments d'ADN sont obtenus comme indiqué ci-après.

Le plasmide pUC8 possédant un site de coupure par AluI proche du site d'insertion EcoRI, on purifie à partir de pAT152 le fragment AluI-AluI de 800 pb constituant la moitié côté EcoRI du fragment (voir fig.5). Ce fragment AluI-AluI est soumis à l'action de l'enzyme BaI31, qui dégrade les extrémités libres de l'ADN double brin. Les nouvelles extrémités ainsi créées pouvant être non franches, le fragment dégradé est traité par le fragment de Klenow de l'ADN polymérase I en présence des quatre désoxynucléotides afin de "remplir" les extrémités simple brin et d'augmenter l'efficacité du clonage. La population de fragments AluI-AluI plus ou moins dégradés a été clonée dans le site SmaI de M13mp10.

La séquence nucléotidique complète du fragment d'ADN EcoRI-AccI de 1086pb contenant le gène de résistance aux lincosamides est présentée sur la figure 6.

La numérotation commence au site EcoRI. La séquence d'amino-acides de la protéine est indiquée. Les séquences -35 et -10 du promoteur et le site de fixation du ribosome sont représentés en caractères gras. Les flèches en traits fins indiquent la séquence répétée de 10 pb en orientation directe. Les flèches en traits épais indiquent la répétition inverse de 26 pb.

b - Identification du gène linA codant pour la résistance aux lincosamides

* recherche du cadre de lecture

La localisation des codons de terminaison (TAG,TGA,TAA,) dans les trois cadres de lecture révèle un seul cadre de lecture ouvert pouvant coder pour une protéine de 21.000 daltons . Il s'étend du codon TAA en position 139 au codon TAA en position 895.On donne sur la figure 7 une représentation graphique des codons d'arrêt de la traduction dans les 3 cadres de lecture des deux brins d'ADN séquencés. Les codons d'initiation de la traduction (ATG, GTG, TTG) sontindiqués par des points dans le seul cadre de lecture ouvert pouvant coder pour le produit du gène linA.

* recherche des signaux de traduction

Les codons initiateurs en phase avec ce cadre de lecture, sont localisés aux positions 154 (TTG),197 (TTG), 218 (ATG), 248 (GTG), 413 (ATG) et 472 (ATG). Cependant seul le codon ATG en position 413 est situé en aval d'une séquence ressemblant à un site de fixation du ribosome. Cette séquence AGTTAGGAGG présente une complémentarité de huit bases sur dix avec l'extrémité 3'-OH de l'ARN ribosomal 16S (3'-OH UCUUUCCUCC 5') de *Bacillus subtilis* et de *B. stearothermophilus*. L'énergie libre d'interaction $\Delta G$ de la structure la plus stable entre cette séquence et l'extrémité 3'-OH de l'ARN 16S est de -16,8 kcl/mole (9). Cette valeur élevée est caractéristique de celles trouvées chez les bactéries à Gram-positif où ce $\Delta G$ varie de -14 à -23 kcl/mole alors que chez *E. coli* sa valeur moyenne est de -9,4 kcal/mole (9). Le choix du codon ATG en position 413 comme codon initiateur est confirmé par une étude statistique suivant le critère de Stormo (10).

Le test statistique de Fickett (11) est utilisé afin de rechercher si la phase de lecture ouverte de 484 pb ainsi localisée est une région codante. Ce test, basé sur la fréquence d'utilisation des codons, permet de discriminer une région codante d'une région non codante. La probabilité obtenue (98%) indique que cette phase de lecture ouverte est une région codante.

* caractéristiques de la protéine

La séquence nucléotidique qui s'étend du codon ATG en position 413 au codon TAA en position 895 peut coder pour une protéine de 161 acides aminés de masse moléculaire 19.020 daltons. Cette valeur est en accord avec celle estimée en gel de polyacrylamide-SDS qui est de 21.000 daltons.

* recherche de signaux de transcription

La structure des promoteurs des gènes issus des bactéries à Gram-positif est généralement très proche de celle du promoteur canonique d'*E. coli* : $TTGACA(X)_{17}TATAAT$ (9,12). La recherche d'homologie littérale avec ce motif nucléotidique permet de déceler un site de reconnaissance putatif pour l'ARN polymérase. Cette séquence $TTGATT(X)_{17}TAGTAT$ est situé à la position 154 (fig 6), c'est-à-dire 250 paires de bases en amont du codon ATG initiateur.

- Cinquate-six paires de bases en aval du codon stop TAA (position 895), on trouve une région pouvant former une structure en boucle (fig.6 et 8) dont l'énergie libre d'interaction est de -24 kcal/mole. Cette boucle est vraisemblablement impliquée dans le contrôle p-indépendant de l'arrêt de transcription (12).

D'autre part, immédiatement après le codon stop (position 895) est située une répétition directe parfaite de 10 pb séparées par 4 pb(fig.6).

EXEMPLE II :

Construction d'une sonde intragénique et utilisation pour la détection de la résistance aux lincosamides chez des souches de staphylocoques et de streptocoques.

On utilise un fragment intragénique *Sau*3A-*Sau*3A de 77pb obtenu par sous clonage dans le bactériophage M13mp8 en opérant comme décrit par Hu et Messing (13).

On étudie par hybridation sur colonies, à l'aide de ce fragment, la distribution du gène linA chez 27 souches de staphylocoques et 4 de streptocoques résistantes à la lincomycine. 16 des souches de staphylocoques et 1 de streptocoques ont montré une homologie avec linA.

Les références bibliographiques dont question ci-dessus sont les suivantes :

1- DATTA,N.,W. HEDGES, D.BECKER, and J.DAVIES.1974. Plasmid-determined fusidic acid resistance in the Enterobacteriaceae. J.Gen.Microbiol. 83:191-196.

2- BACHMANN,B.J. 1983.Linkage map of Escherichia coli K12.Edition 7.Microbiol.Rev. 47:180-230.

3- NOVICK,R.P.,R.C. CLOWES,S.N. COHEN,R.CURTISS,N.DATTA, and S.FALKOW.1976. Univorm nomenclature for bacterial plasmids : a proposal.Bacterial.Rev. 40:168-189.

4- GOTS, J.S. 1945. The detection of penicillinase-producing properties of microorganisms. Science 102:309.

5. BIRNBOIM,H.C. and J.DOLY 1979. A rapid alkaline extraction procedure for screening recombinant plasmid DNA. Nucl. Acids Res. 7:1513-1523.

6. MANIATIS, T.,E.F. FRITSCH and J.SAMBROOK.1982. In Molecular Cloniong, a laboratory manual. Cold Spring Harbor Inc.,New York.

7. RAMBACH, A. and D.S. HOGNESS. 1977.Translation of Drosophila melanogaster sequences in Escherichia coli Proc.Natl.Acad.Sci.USA 74:5041-5045.

8.SANGER,F.S. NICKLEN, and A.R.COULSON. 1977. DNAsequencing with chain-terminating inhibitors.Proc.Natl.Acad.Sci., USA 74:5463-5467.

9. MORAN,CP., N.LANG,SG J. LEGRICE,G.LEE,M.STEPHENS,AL SONENSHEIN, J.PERO and R. LOSIK.1982 Nucleotide sequences that signal the initiation of transcription and translation in Bacillus subtilis. Mol. Gen.Genet.186:339-346.

10. STORMO,D.G.., T.G. SCHNEIDER. and L.M. GOLD. 1982 Characterization of translational initiation sites in E.coli Nucl. Acids Res.10:2971-2996..

11. LECLERCQ, R.,C. CARLIER, J.DUVAL and P.COURVALIN 1985. Plasmidmediated resistance to lincomycin by inactivation in Staphylococcus.Antimicrob.Agents Chemother.

12. ROSENBERG, M. and D. COURT. 1979. Regulatory sequences involved in the promotion and termination of RNA transcription. Annu. Rev. Genet. 13:319-353.

13. HU and MESSING - Elservier Biomedical press. gene 17 (1982) : 271-272.

## REVENDICATIONS

1/ Fragment d'ADN, caractérisé en ce qu'il comprend au moins une partie du gène linA, ce gène codant pour une protéine de résistance aux lincosamides, et étant inclus dans la séquence suivante :

```
GAATTCCTAAG ACGTTACCAC ATTGTTTTAC GTTAAAAGCC TTTTTAATTT TTAAAATTTC   60
 CAGTATATCT GCGTAACTTA CATTATCAAT TTTCTTTTCT CTCCATGGGC GTTGCTTCCC  120
 ACTTTTCGTC ATATCTTTTA ATGTTTCGTT ATCTTGATTT TCGTCAATAT ATTTAGTAGT  180
 ATTATATTGC ATATAAAAAT CCCCCAGTTT TATTTAATGA TTCGACACCT TAATAATATA  240
 ACTGGGGTGT TTTCATGTCA AATTTTCTTT TCAACCACAA AAATACATAA AAACACTGTC  300
 ACATCAACGA TAAATCATAT TTTTAAAAAA GAACGTTATT TCTATATAGT ATCAAGACAA  360
GAAGAAACTC GTTTTCAACT CGTTTCAAAT TCCCAAGTTA GGAGGGATAAA ATG AAA AAT  420
AAT AAT GTA ACA GAA AAA GAA TTA TTT TAT ATT TTA GAT TTA TTT GAA CAC ATG AAA GTA  480
ACT TAT TGG TTA GAT GGT GGC TGG GGG GTA GAT GTA TTA ACT GGA AAA CAA CAA AGA GAA  540
CAC AGA GAT ATA GAT ATA GAT TTT GAC GCT CAA CAC ACT CAA AAA GTT ATA CAA AAA TTA  600
GAA GAT ATA GGA TAC AAA ATA GAA GTT CAT TGG ATG CCT TCA CGT ATG GAA CTT AAG CAT  660
GAA GAA TAT GGG TAT TTA GAT ATT CAT CCT ATA AAT CTA AAT GAT GAT GGA TCA ATT ACC  720
CAA GCA AAC CCA GAA GGT GGT AAT TAT GTT TTC CAA AAT GAC TGG TTT TCA GAA ACT AAT  780
TAC AAA GAT CGA AAA ATA CCA TGT ATT TCA AAA GAA GCT CAA CTT CTT TTT CAT TCT GGT  840
TAT GAT TTA ACA GAA ACA GAC CAT TTT GAT ATA AAA AAT TTA AAA TCA ATA ACA TAA TTG  900
 CTTTATTCCA ATTGCTTTAT TGACGTTGAG CCTCGGAACC CTTAACAATC CCAAAACTTG  960
 TCGAATGGTC GGCTTAATAG CTCACGCTAT GCCGACATTC GTCTGCAAGT TTAGTTAAGG 1020
 GTTCTTCTCA ACTTCAATAA ATTTTCTCGG CATAAATGCG TGTTCTTTTT TGTCTACTTA 1080
 GTAGAC                                                            1086
```

2/ Fragment d'ADN selon la revendication 1, caractérisé en ce qu'il est porté par un plasmide de 2,5kb environ, à savoir pIP855 déposé à la Collection Nationale de Culture de Microorganismes (CNCM) de l'Institut Pasteur le 25 Septembre 1985 sous le n° I-484.

3/ Fragment d'ADN selon la revendication 1, caractérisé en ce qu'il est porté par un fragment d'ADN de restriction EcoRI-AccI de 1,1 kb environ, du plasmide recombinant pAT22, lui-même obtenu par digestion, par EcoRI,de pBR329 de 4,1 kb et de pIP855, puis religation.

4/ Fragment d'ADN selon la revendication 1, caractérisé en ce que la séquence codante du gène linA est définie par un codon d'initiation ATG en position 413 et un codon de terminaison TAG en position 895 dans une phase ouverte de lecture de 482 pb.

5/ Vecteur recombinant comportant au moins une partie d'un fragment d'ADN selon l'une quelconque des revendications 1 à 4.

6/ Procédé d'obtention d'un fragment d'ADN selon l'une quelconque des revendications 1 à 4, caractérisé en ce qu'il comprend :

- la digestion de pAT22 par les endonucléases EcoRI et AccI et religation, ce qui conduit au plasmide recombinant pAT151 comportant un fragment d'ADN de pIP855 de 1,1kb environ,
- le clonage du fragment EcoRI-AccI de 1,1kb de pAT151 dans pUC8 de 2,75 kb avec digestion de pUC8 par AccI et EcoRI et ligation,
- la purification du fragment de 1,1kb, par sous-clonage à partir de pAT152 digéré par EcoRI et PstI.
- le clonage dans une entérobactérie sensible aux antibiotiques MLS, plus spécialement une souche mutante de E.coli sensible aux MLS, en particulier E.coli DB10, en sélectionnant en particulier pour la résistance à l'ampicilline et à la clindamycine et en vérifiant le phénotype de Gots positif des transformants obtenus.

7/ Polypeptide capable d'inactiver les lincosamides et de conférer des propriétés de résistance à la lincomycine, caractérisé en ce qu'il possède une masse moléculaire de l'ordre de 19000 à 21000 daltons, et présente la séquence suivante :

Met Lys Asn

Asn Asn Val Thr Glu Lys Glu Leu Phe Tyr Ile Leu Asp Leu Phe Glu His Met Lys Val

Thr Tyr Trp Leu Asp Glv Gly Trp Gly Val Asp Val Leu Thr Gly Lys Gln Gln Arg Glu

His Arg Asp Ile Asp Ile Asp Phe Asp Ala Gln His Thr Gln Lys Val Ile Gln Lys Leu

Glu Asp Ile Gly Tyr Lys Ile Glu Val His Trp Met Pro Ser Arg Met Glu Leu Lys His

Glu Glu Tyr Gly Tyr Leu Asp Ile His Pro Ile Asn Leu Asn Asp Asp Gly Ser Ile Thr

Gln Ala Asn Pro Glu Gly Gly Asn Tyr Val Phe Gln Asn Asp Trp Phe Ser Glu Thr Asn

Tyr Lys Asp Arg Lys Ile Pro Cys Ile Ser Lys Glu Ala Gln Leu Leu Phe His Ser Gly

Tyr Asp Leu Thr Glu Thr Asp His Phe Asp Ile Lys Asn Leu Lys Ser Ile Thr

8/ Application d'un fragment d'ADN selon l'une quelconque des revendications 1 à 4, plus spécialement d'une séquence intragénique du gène linA pour l'élaboration de sondes de détection de la résistance aux lincosamides dans des cultures cellulaires.

9/ Application selon la revendication 8, caractérisé en ce qu'on utilise comme sonde intragénique un fragment d'ADN de 77pb délimité par les sites de restriction Sau3A-Sau3A.

10/ Utilisation du polypeptide codé par le gène linA pour le dosage de lincosamides isolés ou associés avec d'autres antibiotiques et pour modifier chimiquement les lincosamides en vue par exemple d'hémisynthèse.

11/ Utilisation du peptide codé par le gène linA pour l'élaboration d'antisérum spécifique ainsi que d'anticorps spécifiques, en particulier d'anticorps monoclonaux.

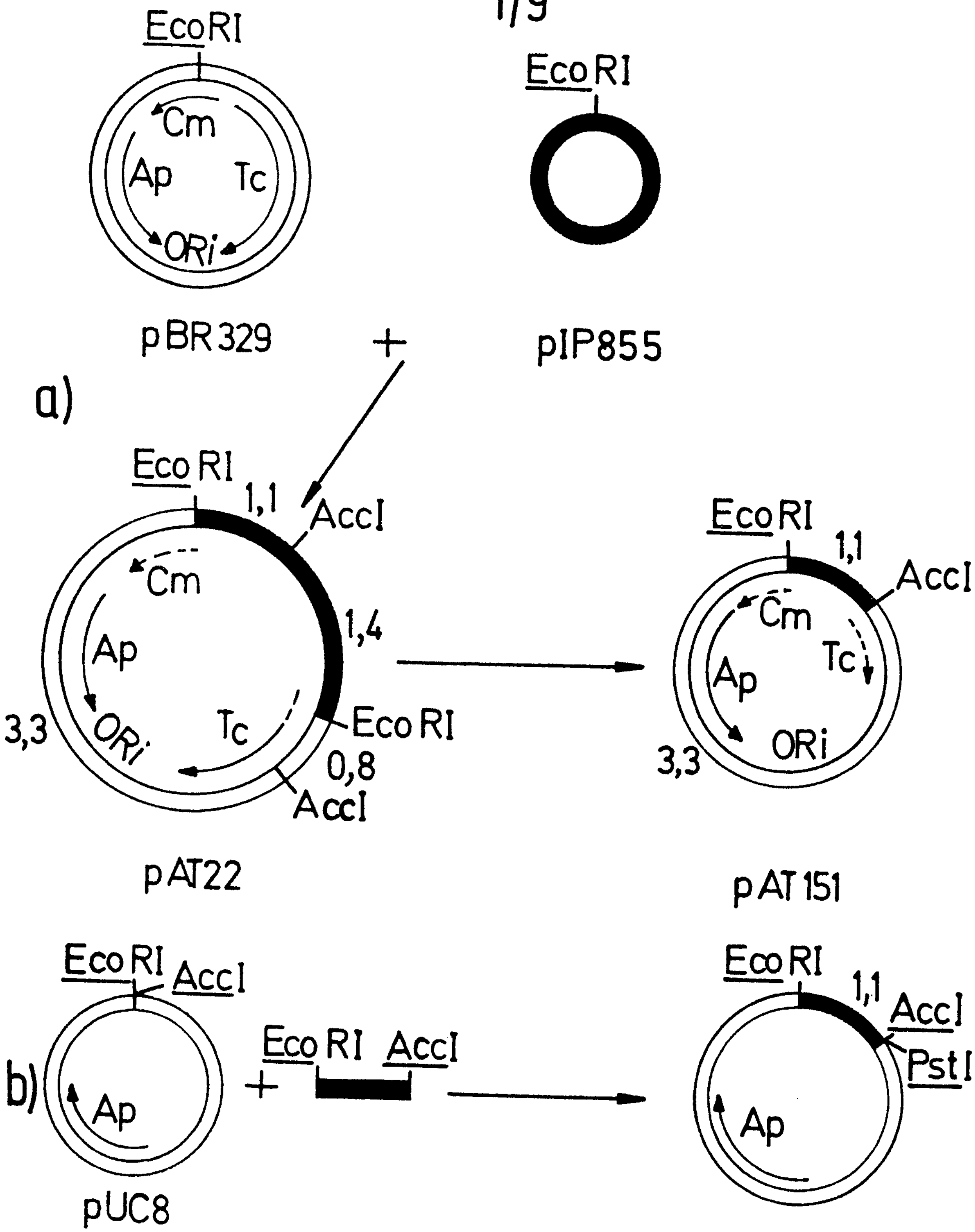
EcoRI
Cm
Ap
Tc
ORi
pBR329
+
EcoRI
pIP855
a)
Eco RI
1,1
AccI
Cm
Ap
1,4
3,3
ORi
Tc
Eco RI
0,8
AccI
pAT22
EcoRI
1,1
AccI
Cm
Tc
Ap
ORi
3,3
pAT151
EcoRI
AccI
b)
Ap
pUC8
+
Eco RI
AccI
EcoRI
1,1
AccI
PstI
Ap
pAT152

FIG.1

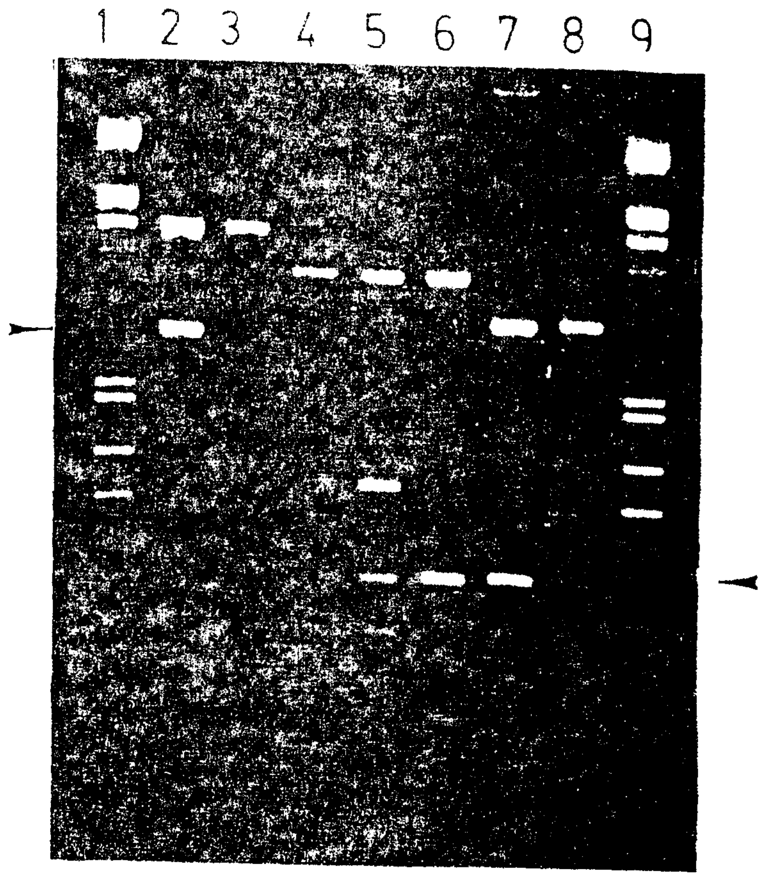


FIG. 2

FIG.3

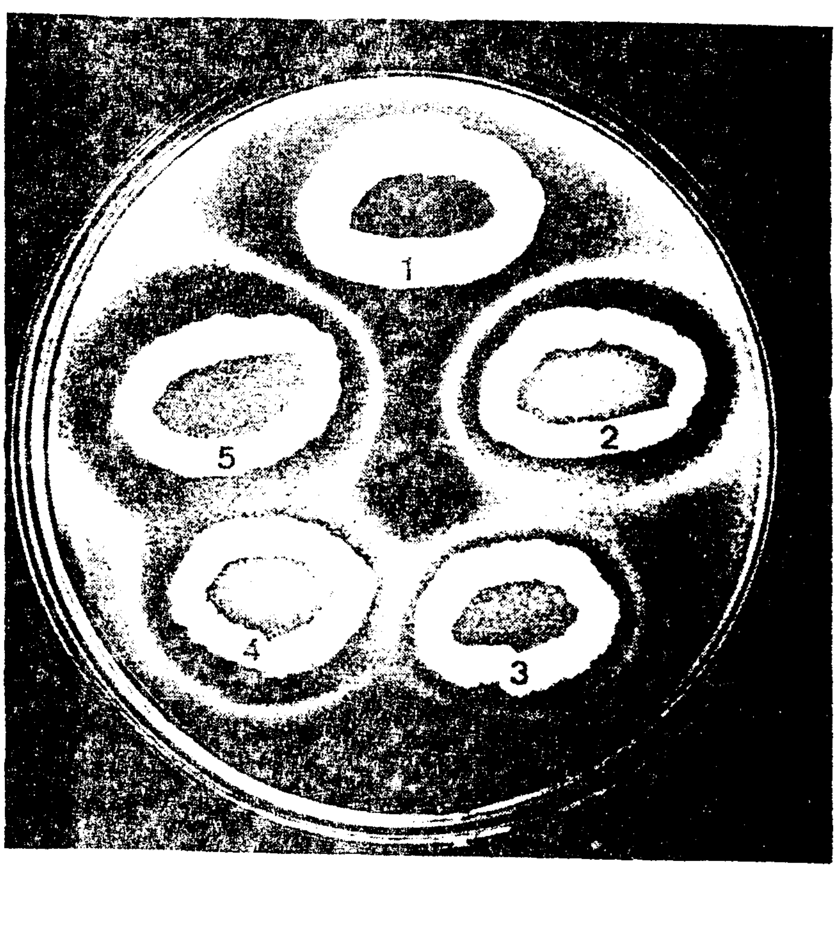

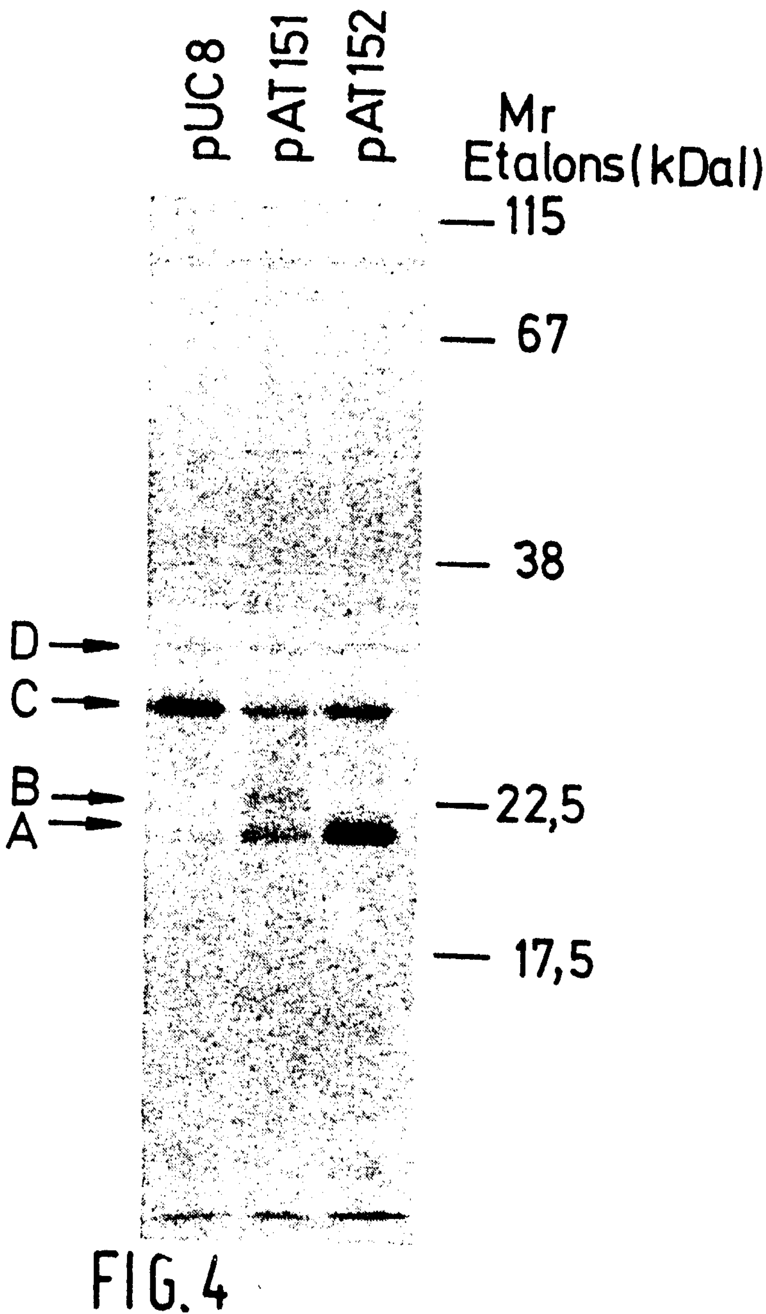


FIG. 4

FIG.5

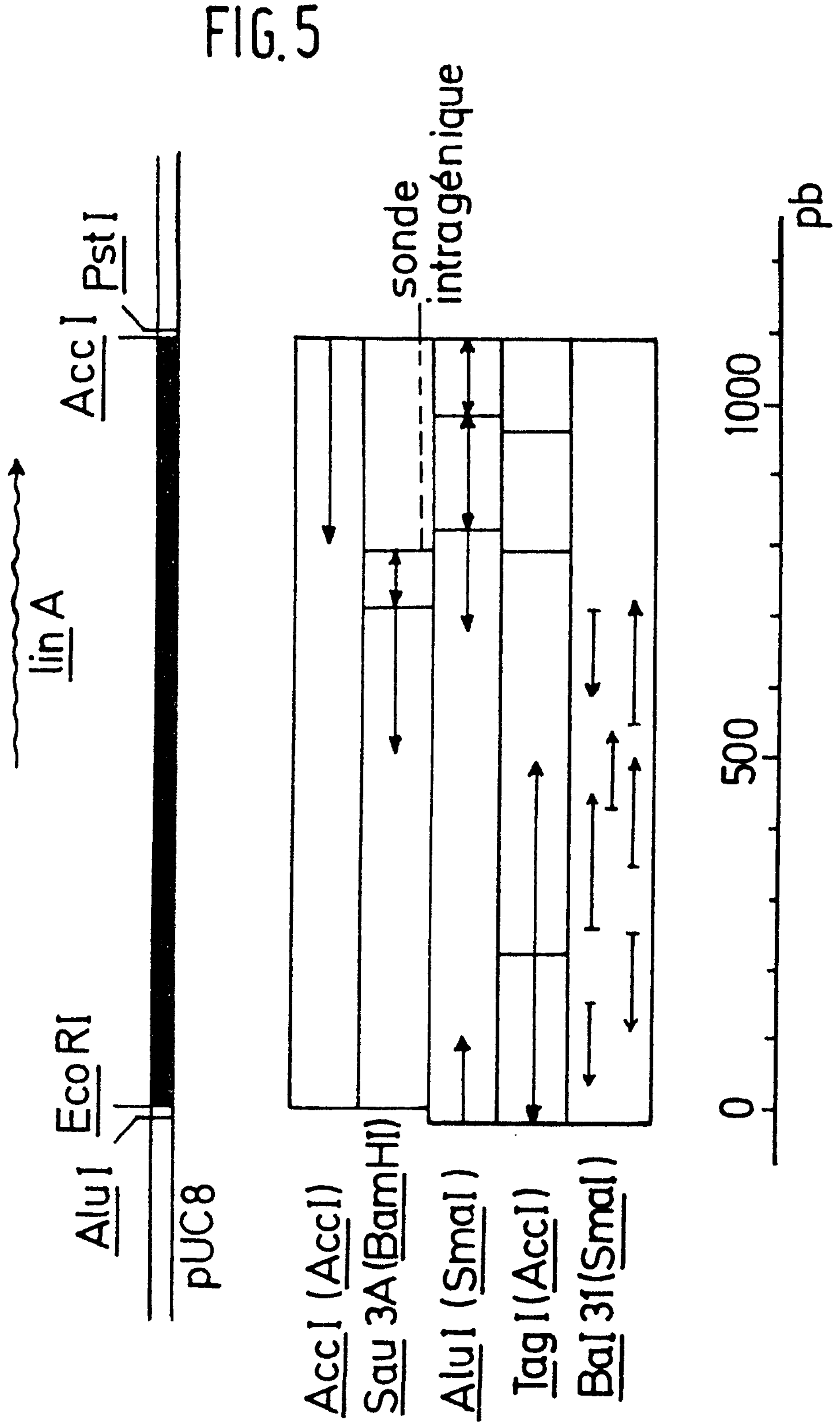
Alu I
Eco RI
Acc I
Pst I
pUC8
lin A
sonde
intragénique
Acc I (Acc I)
Sau 3A (BamHI)
Alu I (Sma I)
Taq I (Acc I)
Bal 31 (Sma I)
0
500
1000
pb

FIG.6A

| | | |
|---|---|---|
| GAATTCCTAAG | ACGTTACCAC | |
| ATTGTTTTAC | GTTAAAAGCC | |
| TTTTTAATTT | TTAAAATTTC | 60 |
| CAGTATATCT | GCGTAACTTA | |
| CATTATCAAT | TTTCTTTTCT | |
| CTCCATGGGC | GTTGCTTCCC | 120 |
| ACTTTTCGTC | ATATCTTTTA | |
| ATGTTTCGTT | ATCTTGATTT | |
| TCGTCAATAT | ATTTAGTAGT | 180 |
| ATTATATTGC | ATATAAAAAT | |
| CCCCCAGTTT | TATTTAATGA | |
| TTCGACACCT | TAATAATATA | 240 |
| ACTGGGGTGT | TTTCATGTCA | |
| AATTTTCTTT | TCAACCACAA | |
| AAATACATAA | AAACACTGTC | 300 |
| ACATCAACGA | TAAATCATAT | |
| TTTTAAAAAA | GAACGTTATT | |
| TCTATATAGT | ATCAAGACAA | 360 |
| GAAGAAACTC | GTTTTCAACT | |
| CGTTTCAAAT | TCCCAAGTTA | |
| GGAGGGATAAA | ATG AAA AAT | 420 |

FIG.6B

| | | | | | | |
|---|---|---|---|---|---|---|
| AAT | AAT | GTA | ACA | GAA | AAA | |
| GAA | TTA | TTT | TAT | ATT | TTA | |
| GAT | TTA | TTT | GAA | CAC | ATG | |
| AAA | GTA | | | | | 480 |
| ACT | TAT | TGG | TTA | GAT | GGT | |
| GGC | TGG | GGG | GTA | GAT | GTA | |
| TTA | ACT | GGA | AAA | CAA | CAA | |
| AGA | GAA | | | | | 540 |
| CAC | AGA | GAT | ATA | GAT | ATA | |
| GAT | TTT | GAC | GCT | CAA | CAC | |
| ACT | CAA | AAA | GTT | ATA | CAA | |
| AAA | TTA | | | | | 600 |
| GAA | GAT | ATA | GGA | TAC | AAA | |
| ATA | GAA | GTT | CAT | TGG | ATG | |
| CCT | TCA | CGT | ATG | GAA | CTT | |
| AAG | CAT | | | | | 660 |
| GAA | GAA | TAT | GGG | TAT | TTA | |
| GAT | ATT | CAT | CCT | ATA | AAT | |
| CTA | AAT | GAT | GAT | GGA | TCA | |
| ATT | ACC | | | | | 720 |

FIG.6C

CAA GCA AAC CCA GAA GGT
GGT AAT TAT GTT TTC CAA
AAT GAC TGG TTT TCA GAA
ACT AAT 780

TAC AAA GAT CGA AAA ATA
CCA TGT ATT TCA AAA GAA
GCT CAA CTT CTT TTT CAT
TCT GGT 840

TAT GAT TTA ACA GAA ACA
GAC CAT TTT GAT ATA AAA
AAT TTA AAA TCA ATA ACA

TAA TTG 900

CTTTATTCCA ATTGCTTTAT
TGACGTTGAG CCTCGGAACC
CTTAACAATC CCAAAACTTG 960

TCGAATGGTC GGCTTAATAG
CTCACGCTAT GCCGACATTC
GTCTGCAAGT TTAGTTAAGG 1020

GTTCTTCTCA ACTTCAATAA
ATTTTCTCGG CATAAATGCG
TGTTCTTTTT TGTCTACTTA 1080

GTAGAC 1086

FIG.7

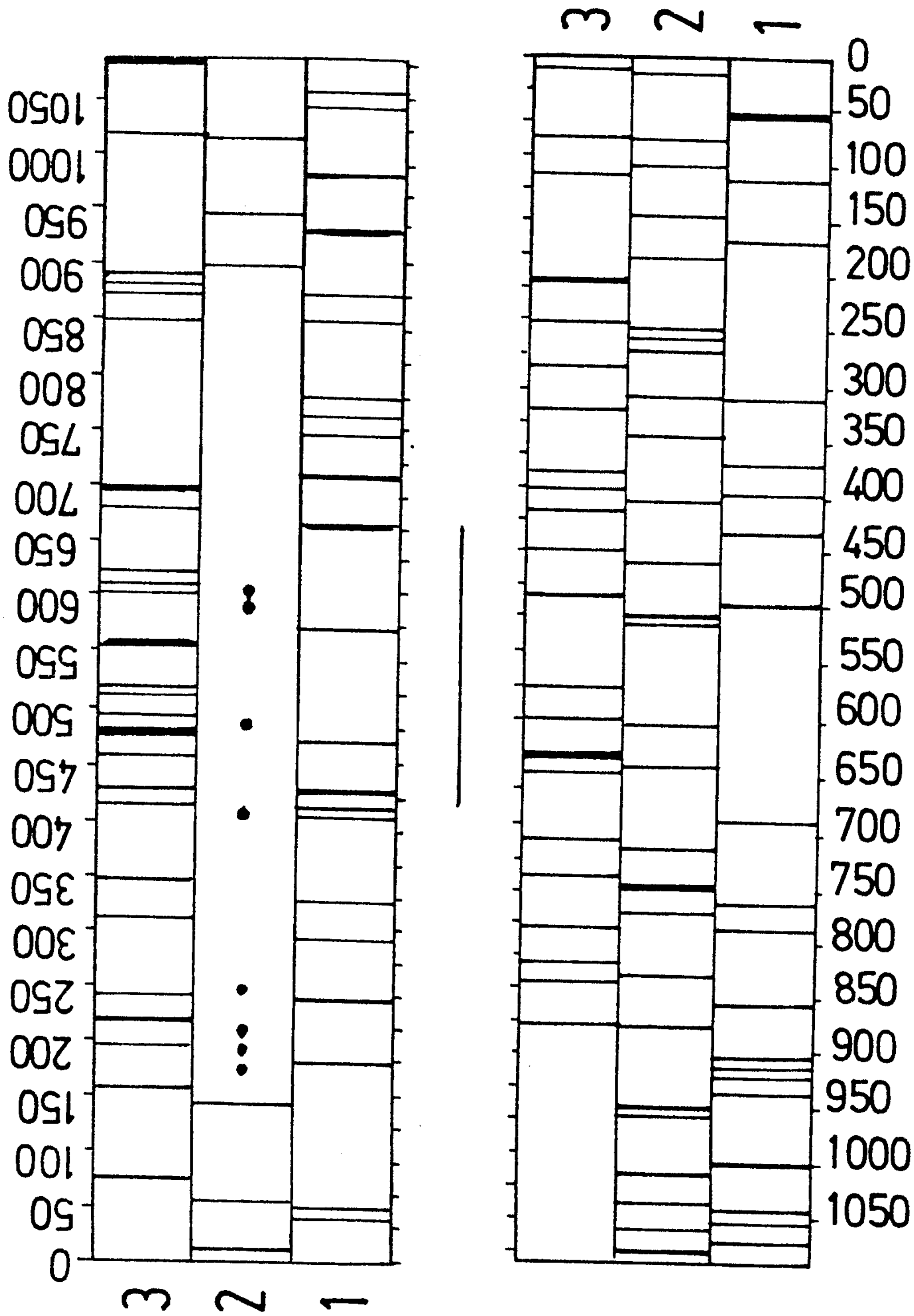
3
2
1
0
50
100
150
200
250
300
350
400
450
500
550
600
650
700
750
800
850
900
950
1000
1050

FIG.8

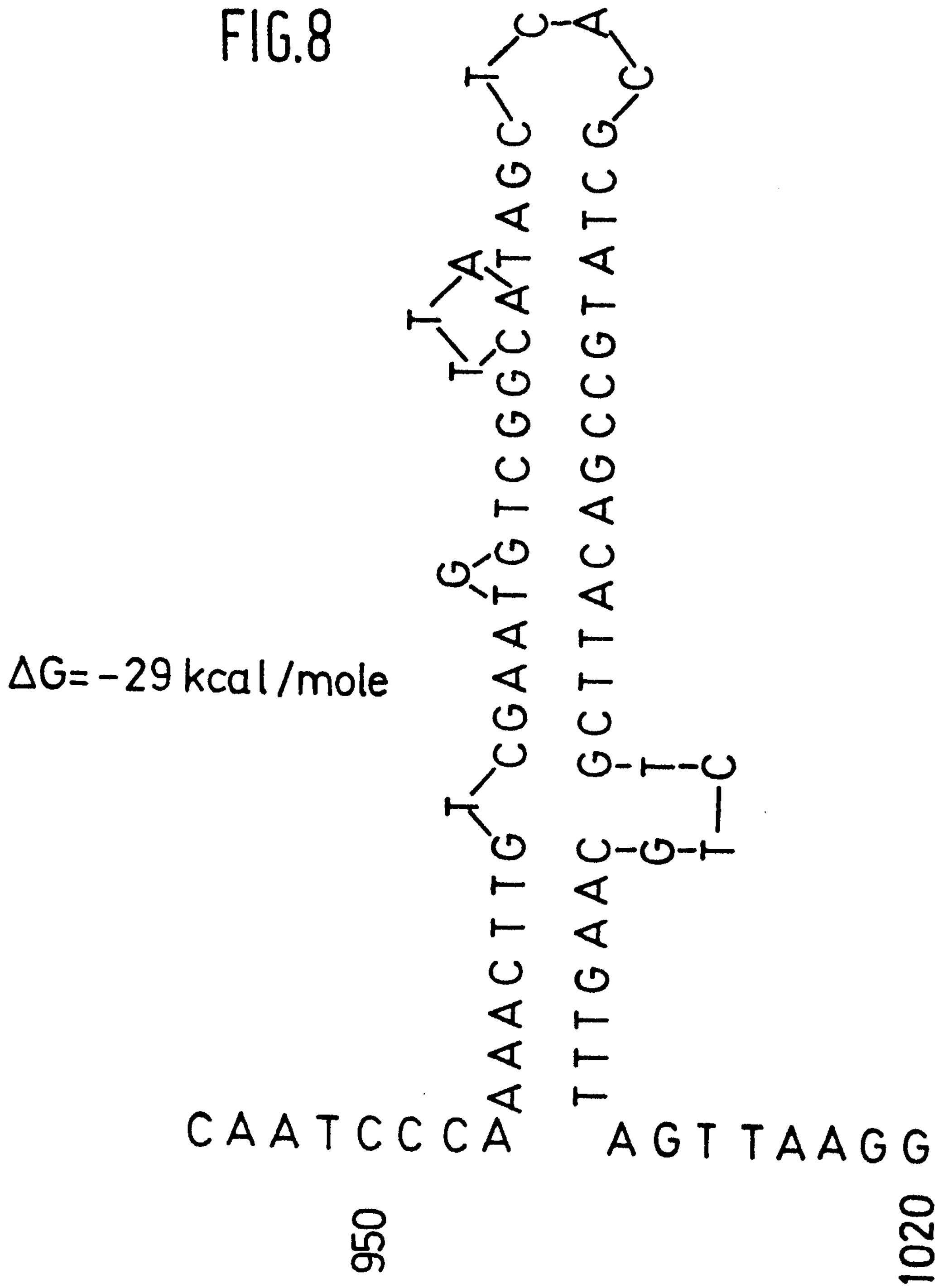

Office européen des brevets

# RAPPORT DE RECHERCHE EUROPEENNE

0219407

Numero de la demande

EP 86 40 2117

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,D | ANTIMICROBIAL AGENTS AND CHEMOTHERAPY, vol. 28, no. 3, septembre 1985, pages 421-424, American Society for Microbiology; R. LECLERCQ et al.: "Plasmid-mediated resistance to lincomycin by inactivation in Staphylococcus haemolyticus" * En entier * | 1-3,5-8 | C 12 N 15/00<br>C 12 N 9/00<br>G 01 N 33/68<br>G 01 N 33/577 |
| A | MICROBIOS LETTERS, vol. 22, 1983, pages 22-29, The Faculty Press, Cambridge, GB; J.A. PEREZ LOPEZ et al.: "Antibiotic resistance mediated by plasmids of a hetero-R strain of Shigella sonnei" * Page 25, tableau 1 * | 1-11 | |
| | | | DOMAINES TECHNIQUES RECHERCHES (Int. Cl.4) |
| | | | C 12 N<br>C 12 P |

Le present rapport de recherche a ete etabli pour toutes les revendications

| Lieu de la recherche | Date d'achevement de la recherche | Examinateur |
|---|---|---|
| LA HAYE | 11-12-1986 | CUPIDO M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82